# EUROPEAN PATENT SPECIFICATION

(11) **EP 1 472 296 B1**
(45) Date of publication and mention of the grant of the patent: **26.03.2008**
(21) Application number: 03702542.6
(22) Date of filing: 28.01.2003
(51) Int. Cl.: C08F 220/24, C08F 222/18, C07C 45/68, C09D 4/00

(54) **FLUORINATED PHOTOINITIATORS IN HIGHLY FLUORINATED MONOMERS**
FLUORIERTE PHOTOINITIATOREN IN HOCHFLUORIERTEN MONOMEREN
PHOTO-INITIATEURS FLUORES DANS DES MONOMERES HAUTEMENT FLUORES

(30) Priority: 04.02.2002 EP 02405071
(43) Date of publication of application: 03.11.2004
(73) Proprietor: Ciba Specialty Chemicals Holding Inc., 4057 Basel (CH)
(72) Inventor: BAUDIN, Gisèle, CH-4123 Allschwil (CH); JUNG, Tunja, 79618 Rheinfelden-Herten (DE)
(86) International application number: PCT/EP2003/000818
(87) International publication number: WO 2003/066693

(56) References cited:
- WO-A-93/12150
- WO-A-94/22925
- US-A- 5 202 359

## Description

The invention relates to novel photoinitiators that are soluble in highly fluorinated monomers.

Fluorinated photoinitiators, which are compatible with highly fluorinated acrylate monomers have been described, for example, in US 5,202,359; in EP 0693087 and in WO93/12150.

However, there is still a need to provide further photoinitiators having a potentially improved solubility in highly fluorinated monomers.

The invention provides photopolymerizable compositions comprising
(A) at least one ethylenically unsaturated photopolymerizable fluocarbon compound selected from polyfluoroalkyl monoacrylates, polyfluoroalkyl monomethacrylates or polyfluoroalkyldiacrylates and
(B) at least one photoinitiator of the formula I 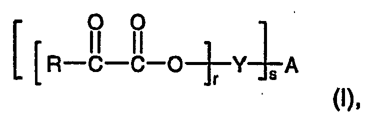
in which
- **R**: is a radical of the formula II 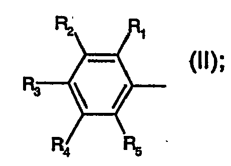 or
- **R**: is naphthyl, anthracyl, phenanthryl or a heterocyclic radical,
the radicals naphthyl, anthracyl, phenanthryl and/or the heterocycle being unsubstituted or substituted by C₁-C₈alkyl, phenyl, OR₆, SR₇ and/or NR₈R₉, where the substituents OR₆, SR₇, NR₈R₉ may form 5- or 6-membered rings via the radicals R₆, R₇, A₈ and/or R₉ with further substituents on the naphthyl, anthracyl or phenanthryl ring or on the heterocycle or with one of the carbon atoms of the naphthyl, anthracyl or phenanthryl ring or with one of the carbon atoms of the heterocycle;
and
R_{1,} R₂, R₃, R₄ and R₅ independently of one another are hydrogen; unsubstituted C₁-C₁₂alkyl or C₁-C₁₂alkyl substituted by OH, C₁-C₄alkoxy, phenyl, naphthyl, halogen, CN and/or -O(CO)R₁₀; or are C₂-Cₗ₂alkyl interrupted by one or more non-successive oxygen atoms; or are OR₆; SR₇; NR₈R₉; halogen; unsubstituted or C₁-C₄alkyl- and/or C₁-C₄alkoxy-substituted phenyl, where the substituents OR₆, SR₇, NR₈R₉ may form 5- or 6-membered rings via the radicals R₆, R₇, R₈ and/or R₉ with further substituents on the phenyl ring or one of the carbon atoms of the phenyl ring;
R₆ and R₇ independently of one another are hydrogen; unsubstituted .
C₁-C₁₂alkyl or C₁-C₁₂alkyl substituted by OH, C₁-C₄alkoxy, phenyl, phenoxy and/or -O(CO)R₁₀; or are C₂-C₁₂alkyl interrupted by one or more non-successive oxygen atoms; or are unsubstituted phenyl, C₃-C₆alkenyl, cyclopentyl, cyclohexyl or naphthyl; or are C₁-C₄alkoxy-, phenyl- and/or C₁-C₄alkyl-substituted phenyl, C₃-C₆alkenyl, cyclopentyl, cyclohexyl or naphthyl;
R₈ and R₉ independently of one another are hydrogen; unsubstituted C₁-Cₗ₂alkyl or C₁-C₁₂alkyl substituted by OH, C₁-C₄alkoxy and/or phenyl; or are C₂-C₁₂alkyl interrupted by one or more non-successive oxygen atoms; or are phenyl, -(CO)R₁₀ or SO₂R₁₁; or
R₈ and R₉, together with the nitrogen atom to which they are attached, form a 5-, 6- or 7-membered ring which is uninterrupted or interrupted by -O- or -NR₁₂-;
R₁₀ is C₁-C₈alkyl; unsubstituted phenyl; or phenyl substituted by C₁-C₄alkyl and/or C₁-C₄alkoxy;
R₁₁ is C₁-C₁₂alkyl, unsubstituted phenyl or phenyl substituted by C₁-C₄alkyl_{;}
R₁₂ is hydrogen; unsubstituted C₁-C₈alkyl; C₁-C₈alkyl substituted by OH or C₁-C₄alkoxy; unsubstituted phenyl; phenyl substituted by OH, C₁-C₄alkyl and/or C₁-C₄alkoxy; and
- s: is a number of 1 to 4;
- r: is a number of 1 or 2;
- Y: is a single bond or is a divalent group C₁-C₁₀alkylene, C₂-C₁₀alkenylene, C₃-C₁₀alkynylene, -(CH₂)ₐ-O-, -[(CH₂)ₐ-O-(CH₂)_{b}]_{c}-, -[(CH₂)ₐ-O]_{q}-(CH₂)_{b}-, -(CH₂)ₐ-O-(CH₂)_{b}-O-, -(CH₂)ₐ-NR₈-(CH₂)_{b}-, -(CH₂)ₐ-O-(CH₂)_{b}-NR₈-(CH₂)_{c}-, -(C₂-C₁₀alkenylene)-O-(CH₂)ₐ-, -(C₂-C₁₀alkynylene)-O-(CH₂)ₐ-, with the proviso that at the site of a bond to the photoinitiator radical 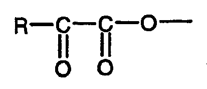 there must always be at least one methylene group;
a is a number from 1 to 10;
b and c independently of one another are a number from 0 to 10; with the proviso that they are, however, at least 1 if the methylene group in question is between two oxygen atoms or one oxygen and one nitrogen atom, and
- q: is a number from 1 to 30.
- **A**: **if s is 1,** is a radical A₀, where A₀ is C₁-C₃₀alkyl, C₁-C₃₀alkenyl, C₁-C₃₀alkynyl or C₁-C₃₀aralkyl, **the radicals being substituted by at least two fluorine** atoms and optionally further substituted by OH-, C₁-C₄alkoxy-, phenyl-, naphthyl-, halogen-, CN-, SR₇-, NR₈R₉- and/or -O(CO)R₁₀-; and the radical A₀ is uninterrupted or interrupted by one or more -O-, -S- or -NR₁₂-;
- **A**: **if s is >1,** is a radical A₁; where A₁ is C₁-C₃₀alkylene, C₁-C₃₀alkenylene, C₁-C₃₀alkynylene or C₁-C₃₀aralkylene, **the radicals being substituted by at least two fluorine** atoms and optionally further substituted by OH-, C₁-C₄alkoxy-, phenyl-, naphthyl-, halogen-, CN-, SR₇, NR₈R₉- and/or -O(CO)R₁₀-; and the-radical A₁ is uninterrupted or interrupted by one or more -O-, -S- or -NR₁₂-.

The residues A₀ and A₁ have preferably fluoralkylchains.

### Definitions

Polyfluoroalkyl monoacrylates are for example C₅F₁₁CH₂OC(O)-CH=CH₂, C₇F₁₅CH₂OC(O)-CH=CH_{2,} C₉F₁₉CH₂OC(O)-CH=CH_{2,} C₈F₁₇SO₂N(CH₃)-(CH₂-CH₂)ₙ-OC(O)-CH=CH₂ and the like.

Polyfluoroalkyl monomethacrylates are for example C₇F₁₅CH₂OC(O)-C(CH₃)=CH₂ C₉F₁₉CH₂OC(O)-C(CH₃)=CH₂, C₁₅F₃₁CH₂OC(O)-C(CH₃)=CH₂, C₁₀F₂₁SO₂N(H)-(CH₂-CH₂)ₙ-OC(O)- C(CH₃)=CH₂, CF₃-CF₂-O-(C₂F₄O)ₘ(CF₂O)nCH₂OCOCH=CH₂ and the like.

Polyfluoroalkyl diacrylates are described in US 5,202,359 and are, for example, CH₂=CH-C(O)-O-CH₂-(CF₂)ₙCH₂-OC(O)-CH=CH₂ n,m = 4-20

C₁-C₃₀Alkyl is linear or branched and is for example C₁-C₃₀, C₁-C₂₄, C₁-C₁₂-, C₁-C₈-, C₁-C₆- or C₁-C₄alkyl. Examples are methyl, ethyl, propyl, isopropyl, n-butyl, sec-butyl, iso-butyl, tert-butyl, pentyl, hexyl, heptyl, 2,4,4-trimethyl-pentyl, 2-ethylhexyl, octyl, nonyl, decyl, undecyl or dodecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl or triacontyl. C₆-C₃₀AIkylene is the corresponding divalent residue.

C₂-C₁₂Alkyl interrupted by one or more oxygen atoms is for example interrupted 1-9, 1-7 or 1 or 2 times by -O-. Where the radicals are interrupted by two or more -O-, the oxygen atoms are each separate from one another by at least one methylene group. This results, for example, in structural units such as -CH₂-O-CH₃, -CH₂CH₂-O-CH₂CH₃, -[CH₂CH₂O]_{y}-CH₃, where y=1-9, -(CH₂CH₂O)₇CH₂CH₃, -CH₂-CH(CH₃)-O-CH₂-CH₂CH₃ or -CH₂-CH(CH₃)-O-CH₂CH₃.

C₁-C₄Alkoxy is likewise linear or branched and is, for example, methoxy, ethoxy, propoxy, isopropoxy, n-butyloxy, sec-butyloxy, iso-butyloxy or tert-butyloxy.

C₃-C₆Alkenyl may be mono- or polyunsaturated and may be linear or branched and is, for example C₃-C₄alkenyl. Examples are allyl, methallyl, 1,1-dimethylallyl, 1-butenyl, 2-butenyl, 1,3-pentadienyl or 1-hexenyl, especially allyl.

C₁-C₃₀Alkenyl is likewise linear or branched and mono- or polyunsaturated and is, for example, vinyl, allyl, butenyl, pentenyl, hexenyl, heptenyl, 2,4,4-trimethyl-pentenyl, 2-ethyl-hexenyl, octenyl, nonenyl, decenyl, undecenyl, dodecenyl, tetradecenyl, pentadecenyl, hexadecenyl, heptadecenyl, octadecenyl, nonadecenyl, icosenyl, henicosenyl, docosenyl, tricosenyl, tetracosenyl, pentacosenyl, hexacosenyl, heptacosenyl, octacosenyl or triacontenyl. C₆-C₃₀Alkenylene is the corresponding divalent residue.

C₁-C₃ₒAlkynyl is linear or branched and mono- or polyunsaturated and is for example ethynyl, propynyl, butynyl, hexynyl, heptynyl, 2,4,4-trimethylpentynyl, 2-ethylhexynyl, octynyl, nonynyl, decynyl, undecynyl, dodecynyl, tetradecynyl, pentadecynyl, hexadecynyl, heptadecynyl, octadecynyl, nonadecynyl, icosynyl, henicosynyl, docosynyl, tricosynyl, tetracosynyl, pentacosynyl, hexacosynyl, heptacosynyl, octacosynyl or triacontynyl. C₆-C₃₀Alkynylene is the corresponding divalent residue.

C₁-C₃₀Aralkyl is alkyl substituted by an aromatic radical. Examples are phenyl-C₁-C₂₄alkyl, naphthyl-C₁-C₂₀alkyl; anthryl-C₁-C₁₆alkyl, phenanthryl-C₁-C₁₆alkyl, the corresponding alkyl radical C₁-C₂₄, C₁-C₂₀, C₁-C₁₆ in each case being substituted by the respective corresponding aromatic radical phenyl, naphthyl, anthryl or phenanthryl. The alkyl radicals are linear or branched and may have the definition indicated above. Examples are benzyl, phenylethyl, a-methyfbenzyl, phenylpentyl, phenylhexyl oder α,α-dimethylbenzyl, especially benzyl, naphthylmethyl, naphthylethyl, naphthylpropyl or naphthyl-1-methylethyl, in particular naphthylmethyl. The alkyl unit may be in either 1 position or 2 position on the naphthyl ring system.

Halogen is fluorine, chlorine, bromine and iodine.

Substituted phenyl is substituted from one to five times, for example once, twice or three times, especially once or twice, on the phenyl ring.

A heterocyclic radical in this context includes not only aliphatic but also aromatic rings containing one or more, especially one or two, heteroatoms. Fused ring systems are included. Examples of suitable heteroatoms include particularly O, N or S. Examples are furyl, thienyl, pyrrolyl, oxinyl, dioxinyl or pyridyl. 5- or 6-membered rings are preferred. As a heterocyclic radical R is for example pyrrolyl, pyrrolidinyl, oxazolyl, pyridinyl, 1,3-diazinyl, 1,2-diazinyl, piperidinyl, morpholinyl, thianthrenyl, furanyl, pyranyl, xanthenyl, imidazolyl thiazoylyl, pyrimidinyl, indazolinyl, indolyl, indazolyl, purinyl, isoquinolyl, quinolyl, xanthyl, thioxanthyl, acridinyl etc.

Where OR₆-, SR₇- or NR₈R₉-substituted naphthyl, anthracyl, phenanthryl or heterocyclic rings form 5- or 6-membered rings via the radicals R₆, R₇, R₈ and/or R₉ with further substituents on the naphthyl, anthracyl or phenanthryl ring or on the heterocycle or with one of the carbon atoms of the naphthyl, anthracyl or phenanthryl ring or with one of the carbon atoms of the heterocycle, this embraces for example the following structures 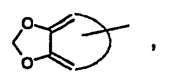 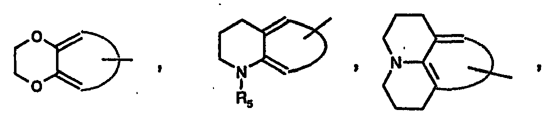 in which the arc and the two double bonds are the aromatic ring system in question.

Where R₁, R₂, R₃, R₄ or R₅ as OR₆, SR₇ or NR₈R₉ form a 5- or 6-membered ring with further substituents on the phenyl ring or with a carbon atom of the phenyl ring, this includes for example the following systems:

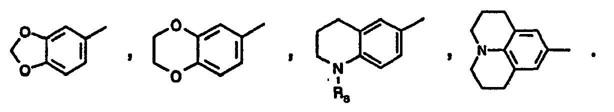

Where R₈ and R₉, together with the nitrogen atom to which they are attached, form a 5- or 6-membered ring which may also be interrupted by -O- or -NR₁₂-, the rings in question are, for example, saturated or unsaturated rings, examples being aziridine, piperazine, pyrrole, pyrrolidine, oxazole, pyridine, 1,3-diazine, 1,2-diazine, piperidine or morpholirie; in particular; morpholinyl, piperidinyl or piperazinyl rings are formed.

With regard to the group Y C₁-C₁₀alkylene is linear or branched alkylene, for example C₁-C₈-, C₁-C₆-, C₁-C₄-, C₂-C₈-, C₂-C₄alkylene, such as methylene, ethylene, propylene, isopropylene, n-butylene, sec-butylene, lso-butylene, tert-butylene, pentylene, hexylene, heptylene, octylene, nonylene or decylene.

In particular, Y is C₁-C₈alkylene, for example ethylene; octylene, 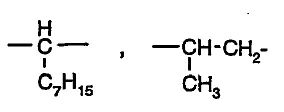 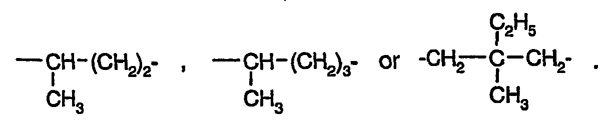 C₃-C₁₀Alkenylene is mono- or polyunsaturated, linear or branched and is for example C₃-C₈-, C₃-C₆-, C₃-C₄alkenylene, for example ethenylene, 1-propenylene, 1-butenylene, 3-butenylene, 2-butenylene, 1,3-pentadienylene, 5-hexenylene or 7-octenylene.

C₂-C₁₀Alkynylene is mono- or polyunsaturated, linear or branched and is for example C₂-C₈-, C₃-C₆-, C₂-C₄alkynylene. Examples are hexynylene, heptynylene, 2,4,4-trimethylpentynylene, 2-ethylhexynylene, octynylene, nonynylene or decynylene.

### Preferred photoinitiators

Preferably Y is a bond.

R in particular is a radical of the formula II or is naphthyl, and is preferably a radical of the formula II.

R₁, R₂, R₃, R₄ and R₅ are especially hydrogen, C₁-C₄alkyl or C₁-C₄alkoxy. Preferably all of R₁-R₅ are hydrogen.

R₆ and R₇ are especially hydrogen, C₁-C₄alkyl; unsubstituted or C₁-C₄alkyl- and/or C₁-C₄alkoxy-substituted phenyl, or -O-interrupted C₂-C₈alkyl , preferably C₁-C₄alkyl or hydrogen.

R₈ and R₉ are especially C₁-C₄alkyl, preferably methyl, or together with the nitrogen atom to which they are attached form a morpholinyl radical.

R₁₀ is especially C₁-C₄alkyl or phenyl.

R₁₁ is preferably C₁-C₄alkyl or phenyl.

R₁₂ is preferably hydrogen, C₁-C₄alkyl or OH-substituted C₁-C₄alkyl.

A₀ is in particular a C₆-C₃₀alkyl radical which is substituted by seven or more fluorine atoms. Examples are -(CH₂)₂-(CF₂)₅-CF₃, -(CH₂)₂-(CF₂)₆ -CF(CF₃)₂, -CH₂-(CF₂)₆-CF₃, -(CH₂)₂-(CF₂)₇-CF₃, -CH₂-(CF₂)₁₂-CF₃, -CH₂-(CF₂)₅-CHF₂,-CH₂-(CF₂)₇-CH₂-OH.

A₁ is preferably a divalent C₆-C₃₀alkylene radical which is substituted by seven or more fluorine atoms. Examples are: -CH₂-(CF₂)₇-CH₂-, -CH₂-(CF₂)₈-CH₂-, -CH₂-CH(CH₂-C₆F₁₃)-.

Preference is given to compounds of the formula I, wherein
- **R**: is a radical of the formula II in which R₁, R₂, R₃, R₄ and R₅ independently of one another are hydrogen or C₁-C₄alkyl or C₁-C₄alkoxy;
- **Y**: is a single bond,
- **A**: **if s is 1,** is a radical A₀, A₀ being C₆-C₃₀alkg substituted by seven or more fluorine atoms and optionally further substituted by OH.
- **A**: **if s is >1,** is a radical A₁; A₁ being C₆-C₃₀alkylene substituted by seven or more fluorine atoms and optionally further substituted by OH.

Particular preference is given to compounds of the formula I wherein
- **R**: is phenyl,
- **Y**: is a single bond;
- **A**: **if s is 1,** is a radical A₀, Ao being are -(CH₂)₂-(CF₂)₅-CF₃, -(CH₂)₂-(CF₂)₆ -CF(CF₃)₂, -CH₂-(CF₂)₆-CF₃, -(CH₂)₂-(CF₂)₇-CF₃, -CH₂-(CF₂)₁₂-CF₃, -CH₂-(CF₂)₅-CHF₂, -CH₂-(CF₂)₇-CH₂-OH
- **A,**: **if s is >1,** is a radical A₁;A₁ being : -CH₂-(CF₂)₇-CH₂-, -CH₂--(CF₂)₈-CH₂-, -CH₂-CH(CH₂-C₆F₁₃)-.

### Preparation

The compounds of the formula I are prepared by customary methods known to the person skilled in the art.
I. For example, they may be obtained by esterifying the corresponding glyoxalic acid compound under esterification conditions common in the art, for example in the presence of an acid or of coupling reagents of the Mitsunobu type, e.g. N,N'-dicyclohexylcarbodiimide (DCC) or 1,1'-carbonyldiimidazole (CDI) (coupling reagents of the Mitsunobu type are well known to the person skilled in the art and are described, for example, in "Progress in the Mitsunobu reaction. A review. Org. Prep. Proced. Int. (1996), 28(2), 127-64" or "The Mitsunobu reaction. Org. React. (N. Y.) (1992), 42 335-656"): 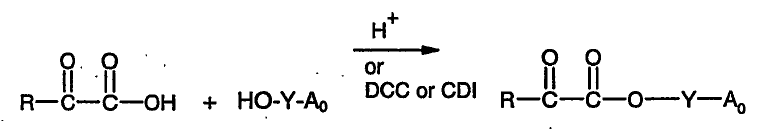 or 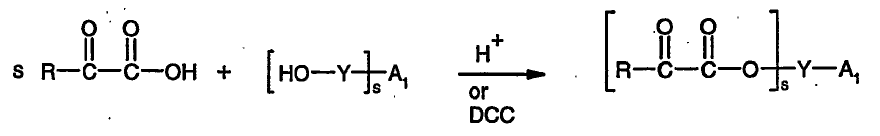 s, R, Y A₀ and A₁ have the definition indicated above.
II. Another possibility of preparing the compounds of the formula I which contain no siloxane radical is the reaction of alcohols with glyoxalic esters, such as the corresponding methyl ester, in the presence of a catalyst: 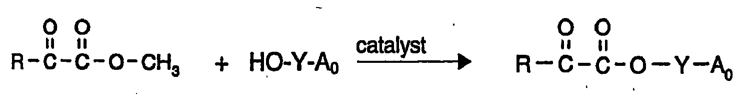 or 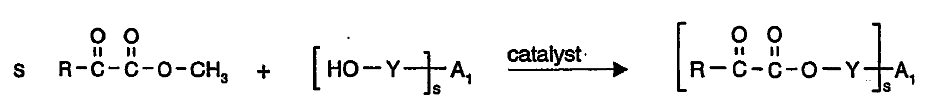 R, s, A₀ and A₁ have the definition indicated above and Y is a bond. Such compounds were described, for example, by Neckers et al in Tetrahedron 53 (21) (1997) 7165 or are part of the teaching of US 4,024,297.
   The catalysts used include, for example, the catalysts well known to the person skilled in the art for transesterification reactions, such as dibutyltin oxide or p-toluenesuffonic acid or a base.
III. Another possibility of obtaining the compounds of the invention having a radical A₀ or A₁ is the base-catalyzed reaction of arylglyoxaloyl halides, preferably the chlorides, with an alcohol: 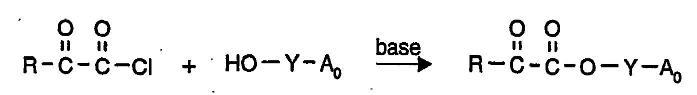 or 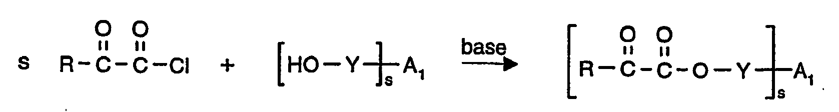 R, s, Y, A₁ and A₀ have the definitions described above.
   The bases to be used for such reactions are well known to the person skilled in the art. Aqueous bases must not be used. Examples of suitable bases are carbonates, tert-amine bases, such as triethylamine, or pyridine.
IV. Furthermore, the compounds of the invention having radicals A₀ or A₁ may be obtained, for example, by reacting alcohols with corresponding arylacetic esters in the presence of a catalyst, with subsequent oxidation: 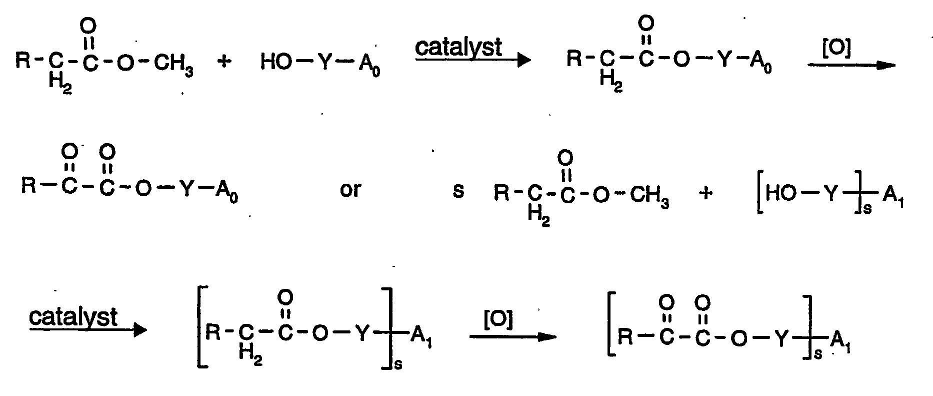 R, s, Y, A₁ and A₀ have the definitions described above.
   The catalysts used are, for example, the catalysts which are well known to the person skilled in the art for transesterification reactions, such as dibutyltin oxide or p-toluenesulfonic acid.
   The oxidation step may take place, for example, as described in J. Chem. Soc. Chem. Comm. (1993), 323 or in Synthesis (1994), 915*.*
V. An example of a further suitable method of preparing the compounds of the invention of the formula I having a radical A₀ or A₁ is the reaction of corresponding hydroxy-substituted arylacetic esters with alcohols, with subsequent oxidation: 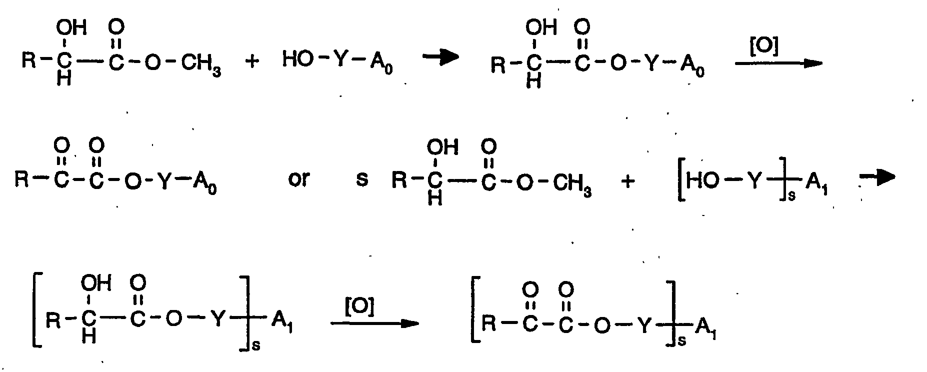 R, s, Y,A₁ and A₀ have the definitions described above.
   The oxidation may be carried out, for example, in accordance with the method described in J. Chem. Soc. Chem. Comm. (1994), 1807*.*
VI. A further preparation possibility for the compounds of the formula I of the invention having a radical A₀ or A₁ is the acid-catalyzed reaction of arylcarboxylic cyanides with alcohols: 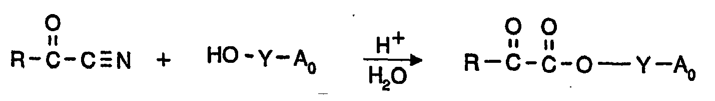 or 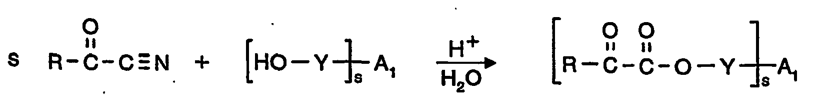 R, s, Y, A₀ and A₁ have the definitions described above.
VII. The compounds of the formula I of the invention having a radical A₀ or A₁ may also be obtained, for example, by Friedel-Crafts reaction of aryls with oxocarboxylic chlorides in the presence of aluminium chloride: 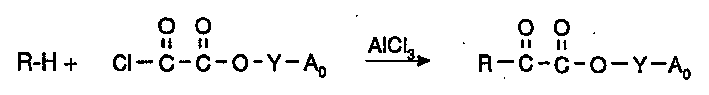 or 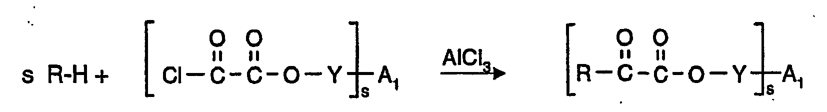 R, s, Y, A₀ and A₁ have the definitions indicated above.

Catalysts which can be used are the customary catalysts, well known to the person skilled in the art, for Friedel-Crafts reactions, examples being tin chloride, zinc chloride, aluminium chloride, titanium chloride or acidic earths.

In the preparation of asymmetric compounds of the formula I, i.e. those in which s is ≥ 2 and the different groups R each have different meanings, the reaction is carried out using the corresponding different starting materials, appropriately in a ratio of 1:1.

The reactions I, II, IV and V may generally be carried out without using a solvent, with one of the liquid reaction components, for example the alcohol, serving as solvent. It is, however, also possible to conduct the reactions in an inert solvent. Suitable solvents are, for example, aliphatic or aromatic hydrocarbons such as, for example, alkanes and alkane mixtures, cyclohexane, benzene, toluene or xylene. The boiling point of these solvents should, though, be above that of the alcohol which forms during the reaction.

The other syntheses set out above are appropriately conducted in an inert solvent. Suitable examples include those indicated above. In the case of reaction I it is appropriate to ensure that the water which forms during the reaction is removed from the reaction mixture. In the case of reactions II, IV and V it is appropriate to ensure that the alcohol which forms during the reaction is removed from the reaction mixture. This is done, for example, by distillation. The reactions are conducted at different temperatures depending on the solvents and starting materials used. The temperatures and other reaction conditions required for the corresponding reactions are common knowledge and are well known to the person skilled in the art.

The reaction products may be separated and purified by customary methods, such as by crystallization, distillation or chromatography.

The preparation of the starting materials required for the synthesis of the compounds of the formula I of the invention is common knowledge and is well known to the person skilled in the art. Indeed, some of the compounds are available commercially.

For example, the arylglyoxalic esters are obtained by Friedel-Crafts reaction from the aryls and the corresponding oxocarboxylic methyl ester chloride, or by esterification of arylglyoxaloyl chlorides with alcohols.

Arylglyoxaloyl chlorides may be obtained, for example, by chlorination of the corresponding acid using, for example, SOCl₂. Arylcarboxylic cyanides may be obtained, for example, by reacting the corresponding acid chlorides with CuCN.

Arylacetic methyl esters are preparable, for example, by acid-catalyzed reaction of aryl-CH₂-CN with methanol. This reaction is described, for example, in Org. Syn. Coll. Vol. I, 270*.* The corresponding aryl-CH₂-cyanides may be obtained, for example, from the corresponding chlorides using NaCN, as disclosed, for example, in Org. Syn. Coll. Vol. I, 107 and Org. Syn. Coll. Vol IV, 576*.*

The synthesis of arylacetic ethyl esters can be found, for example, in J. Chem. Soc. Chem. Comm. (1969), 515*,* where the corresponding aryl bromide is reacted with N₂CH₂COOC₂H₅ in the presence of Li/diethyl ether, CuBr. Another method, the reaction of aryl bromides with ethyl acetate and NaH, is described, for example, in J. Am. Chem. Soc. (1959) 81, 1627*.* J. Org. Chem. (1968) 33, 1675 sets out the Grignard reaction of aryl bromides with BrCH₂COOC₂H₅ to give the arylacetic ethyl ester.

The preparation of the alcohols is well known to the person skilled in the art and is widely described in the literature. Many of these compounds are available commercially.

The preparation of the photoinitiator starting materials which in accordance with the invention are modified with A or A₀ is known to the person skilled in the art and is performed in accordance with customary methods. The preparation of the glyoxalic ester initiators, for example, is described in US 4,475,999, US 4,038,164, EP 132868, GB 1534320, US 4,279,718, US 4,308,394, US 3,930,868 and WO 98/33761.

In accordance with the invention, the photoinitiators are used to cure free-radically polymerizable systems, the objective being to obtain a hardened surface having outstanding properties. The present invention therefore provides both, the use of the photoinitiators of the formula I in purely photocurable formulations and the use of the photoinitiators of the formula I in mixed photochemically and thermally curable formulations. Thermal curing may take place before, during or after exposure to light.

Thus, the invention provides a process for producing coatings having stable scratch-resistant surfaces, in which
(1) a photocurable composition as defined above is prepared;
(2) this composition is applied to a substrate; and
(3) the composition is cured either only by exposure to electromagnetic radiation with a wavelength ranging from 200 nm into the IR region, or by exposure to electromagnetic radiation with a wavelength ranging from 200 nm into the IR region and prior, simultaneous and/or subsequent exposure to heat.

The term "from 200 nm into the IR region" denotes from 200 nm to 2500 nm, in particular from 200 nm to 1000 nm.

The term "and/or" is intended to denote that it is possible for not only one of the defined alternatives (substituents) to be present but also two or more different alternatives (substituents) of those defined together, i.e. mixes of different alternatives (substituents).

The term "at least" is intended to define one or more than one, for example one or two or three, preferably one or two.

The invention accordingly also provides a process as described above in which the photocurable formulation comprises as a further component at least one thermally crosslinkable compound (C) and the formulation is cured by exposure to light whose wavelength extends from 200 nm into the IR region and by prior, simultaneous and/or subsequent exposure to heat.

In accordance with the invention, the compounds of the formula I may be used as photoinitiators for the photopolymerization of ethylenically unsaturated fluorcarbon compounds or mixtures comprising such compounds.

The invention also provides a method of causing a photoinitiator comprising ethylenically unsaturated photopolymerizable fluocarbon compounds selected from polyfluoroalkyl monoacrylates, polyfluoroalkyl monomethacrylates or polyfluoroalkyldiacrylates and which comprises adding a photoinitiator of the formula I to the photopolymerizable mixture comprising the ethylenically unsaturated photopolymerizable fluocarbon compounds.

The photoinitiators may also be used in combination with other photoinitiators (E) and/or further additives (D).

The above photopolymerizable composition may be used in dual-cure systems.

Thus, the invention further provides photopolymerizable compositions comprising
(A) at least one ethylenically unsaturated photopolymerizable fluocarbon compound selected from polyfluoroalkyl monoacrylates, polyfluoroalkyl monomethacrylates or polyfluoroalkyldiacrylates and
(B) at least one photoinitiator of the formula I, and
(C) at least one fluor modified resin which is thermally crosslinkable.

In accordance with the invention, the compositions may also comprise further, different photoinitiators (E) and/or further additives (D). The addition of thermal crosslinking catalysts is also possible. Suitable examples are set out later on below.

Coatings containing fluorinated photoinitiators show good surface properties like chemical resistance, scratch resistance, adhesion to polymer matrixes and have good transparency.

Said coatings are suitable for optical material because of its high transparency and low refractive index.

It is also possible to add binders to the compositions of the invention, which is especially appropriate when the photopolymerizable compounds are liquid or viscous substances. The amount of the binder can be for example 5-95, preferably 10-90 and especially 40-90% by weight, based on the overall solids. The choice of binder is made depending on the field of use and the properties required for that field, such as developability in aqueous and organic solvent systems, adhesion to substrates, and oxygen sensitivity, for example.

Suitable binders are fluorinated arylates or methacrylates or mixtures thereof.

Besides the photoinitiator, the photopolymerizable mixtures may comprise various additives (D). Examples of these are thermal inhibitors, which are intended to prevent premature polymerization, such as hydroquinone, hydroquinone derivatives, p-methoxyphenol, β-naphthol or sterically hindered phenols such as 2,6-di(tert-butyl)-p-cresol. To increase the dark storage stability it is possible to use copper compounds, such as copper naphthenate, copper stearate or copper octoate, phosphorus compounds, such as triphenylphosphine, tributylphosphine, triethyl phosphite, triphenyl phosphite or tribenzyl phosphite, quaternary ammonium compounds, such as tetramethylammonium chloride or trimethylbenzylammonium chloride, or hydroxylamine derivatives, such as N-diethylhydroxylamine, for example. For the purpose of excluding atmospheric oxygen in the course of the polymerization it is possible to add paraffin or similar wax-like substances, which at the beginning of polymerization migrate to the surface, on account of their poor solubility in the polymer, where they form a transparent surface layer which prevents the ingress of air. Similarly, it is possible to apply an oxygen-impermeable layer. Light stabilizers which can be added include UV absorbers, such as those of the hydroxyphenylbenzotriazole, hydroxyphenylbenzophenone, oxalmide or hydroxyphenyl-s-triazine type. These compounds may be used individually or in mixtures, with or without the use of sterically hindered amines (HALS).

Examples of such UV absorbers and light stabilizers are
1. 2-(2'-Hydroxyphenyl)benzotriazoles, for example 2-(2'-hydroxy-5'-methylphenyl)-benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chloro-benzotriazole, 2-(3'-tert-butyl- 2-hydroxy-5"-methylphenyl)-5-chloro-benzotriazole, 2-(3'-sec-butyl-5'-tert-butyl-2'-hydroxyphenyl)-benzotriazole, 2-(2'-hydroxy-4'-octyloxyphenyl)benzotriazole, 2-(3',5'-di-tert-amyl-2'-hydroxyphenyl)benzotriazole, 2-(3',5'-bis-(α,α-dimethylbenzyl)-2'-hydroxyphenyl)-benzotriazole, 2-(3'-tert-butyl-2'-hydroxy-5'-(2-octyloxycarbonylethyl)phenyl)-5-chlorobenzotriazole, 2-(3'-tert-butyl-5'-[2-(2-ethylhexyloxy)-carbonylethyl]-2'-hydroxyphenyl)-5-chloro-benzotriazole, 2-(3'-tert-butyl-2'-hydroxy-5'-(2-methoxycarbonylethyl)phenyl)-5-chloro-benzotriazole, 2-(3'-tert-butyl-2'-hydroxy-5'-(2-methoxycarbonylethyl)phenyl)-benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole, 2-(3'-tert-butyl-2'-hydroxy-5'-(2-isooctyloxycarbonylethyl)phenylbenzotriazole, 2,2'-methylene-bis[4-(1,1,3,3-tetramethylbutyl)-6-benzotriazol-2-ylphenol]; the transesterification product of 2-[3'-tert-butyl-5'-(2-methoxycarbonylethyl)-2'-hydroxyphenyl]-2H-benzotriazole with polyethylene glycol 300; [R-CH₂CH₂-COO-CH₂CH₂]₂- where R = 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)-phenyl]benzotriazole; 2-[2'-hydroxy-3'-(1,1,3,3-tetramethylbutyt)-5'-(α,α-dimethylbenzyl)-phenyl]benzotriazole.
2. 2-Hydroxybenzophenones, for example the 4-hydroxy, 4-methoxy, 4-octyloxy, 4-decyloxy, 4-clodecyloxy, 4-benzyloxy, 4,2',4'-trihydroxy and 2'-hydroxy-4,4'-dimethoxy derivatives.
3. Esters of substituted and unsubstituted benzoic acids, for example 4-tert-butyl-phenyl salicylate, phenyl salicylate, octylphenyl salicylate, dibenzoyl resorcinol, bis(4-tert-butylbenzoyl) resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate.
4. Acrylates, for example ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxy-cinnamate, methyl α-carbomethoxy-p-methoxycinnamate and N-(β-carbomethoxy-β-cyanovinyl)-2-methylindoline.
5. Sterically hindered amines, for example bis(2,2,6,6-tetramethyl-4-piperidyl) sebacate, bis(2,2,6,6-tetramethyl-4-piperidyl) succinate, bis(1,2,2,6,6-pentamethyl-4-piperidyl) sebacate, bis(1-octyloxy-2,2,6,6-tetramethyl-4-piperidyl) sebacate, bis(1,2,2,6,6-pentamethyl-4-piperidyl) n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl) 1,2,3,4-butane-tetracarboxylate, 1,1'-(1,2-ethanediyl)-bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidyl) 2-n-butyl-2-(2-hydroxy-3,5-di-tert-butylbenzyl)-malonate, 3-n-octyl-7,7,9,9-tetramethyl-1,3,8-triazaspiro[4.5]decane-2,4-dione, bis(1-octyloxy-2,2,6,6-tetramethylpiperidyl) sebacate, bis(1-octyloxy-2,2,6,6-tetramethylpiperidyl) succinate, linear or cyclic condensates of N,N'-bis-(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)-1,3,5-triazine and 1,2-bis-(3-aminopropylamino)-ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensation product of N,N'-bis(2,2,6,6-tetramethyl-4-piperidyl)hexamethylenediamine and 4-cyclohexylamino-2,6-dichloro-1,3,5-triazine, a condensation product of 1,2-bis(3-aminopropylamino)ethane and 2,4,6-trichloro-1,3,5-triazine as well as 4-butylamino-2,2,6,6-tetramethylpiperidine (CAS Reg. No.[136504-96-6]); N-(2,2,6,6-tetramethyl-4-piperidyl)-n-dodecylsuccinimide, N-(1,2,2,6,6-pentamethyl-4-piperidyl)-n-dodecylsuccinimide, 2-undecyl-7,7,9,9-tetramethyl-1-oxa-3,8-diaza-4-oxospiro[4.5]decane, a reaction product of 7,7,9,9-tetramethyl-2-cycloundecyl-1-oxa-3,8-diaza-4-oxospiro [4.5]decane und epichlorohydrin, 1,1-bis(1,2,2,6,6-pentamethyl-4-piperidyloxycarbonyl)-2-(4-methoxyphenyl)ethene, N,N'-bis-formyl-N,N'-bis(2,2,6,6-tetramethyl-4-piperidyl)hexamethylenediamine, diester of 4-methoxy-methylene-malonic acid with 1,2,2,6,6-pentamethyl-4-hydroxypiperidine, poly[methylpropyl-3-oxy-4-(2,2,6,6-tetramethyl-4-piperidyl)]siloxane, reaction product of maleic acid anhydride-α-olefincopolymer with 2,2,6,6-tetramethyl-4-aminopiperidine or 1,2,2,6,6-pentamethyl-4-aminopiperidine.
6. Oxamides, for example 4,4'-dioctyloxyoxanilide, 2,2'-diethoxyoxanilide, 2,2'-dioctyloxy5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxy-disubstituted oxanilides and mixtures of o- and p-ethoxy-disubstituted oxanilides.
7. 2-(2-HydroxyDhenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxy-propoxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine, 2-[4-(dodecyloxy/tridecyloxy-2-hydroxypropoxy)-2-hydroxy-phenyl]-4,6-bis(2,4-dimethylphenyl)-1,3,5-triazine, 2-[2-hydroxy-4-(2-hydroxy-3-dodecyloxy-propoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxy-propoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxypropyloxy]phenyl}-4,6-bis(2,4-dimethylphenyl)-1,3,5-triazine.
8. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, bis(2,6-di-tert-butyl-4-methylphenyl) pentaerythritol diphosphite, diisodecyloxypentaerythritol diphosphite, bis(2,4-di-tert-butyl-6-methylphenyl) pentaerythritol diphosphite, bis(2,4,6-tris(tert-butyl) phenyl pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenzo[d,g]-1,3,2-dioxaphosphocin, bis(2,4-di-tert-butyl-6-methylphenyl) methyl phosphite, bis(2,4-di-tert-butyl-6-methylphenyl) ethyl phosphite, 6-fluoro-2,4,8,10-tetra-tert-butyl-12-methyl-dibenzo[d,g]-1,3,2-dioxaphosphocin, 2,2',2"-nitrilo[triethyl tris(3,3',5,5'-tetra-tert-butyt-1,1-biphenyl-2,2'-diyl)phosphite], 2-ethylhexyl-(3,3',5,5'-tetra-tert-butyl-1,1'-biphenyl-2,2'-diyl )phosphite, 5-butyl-5-ethyl-2-(2,4,6-tri-tert-butylphenoxy)-1,3,2-dioxaphosphirane.

Furthermore, it is possible to use additives customary in the art, such as antistatics, flow improvers and adhesion promoters.

In order to accelerate the photopolymerization it is possible to add, as further additives (D), amines, such as triethanolamine, N-methyldiethanolamine, ethyl p-dimethylaminobenzoate, or Michler's ketone. The effect of the amines may be boosted by adding aromatic ketones of the benzophenone type. Examples of amines which can be used as oxygen scavengers are substituted N,N-dialkylanilines, as described in EP-A 339841. Further accelerators, coinitiators and autoxidizers are thiols, thioethers, disulfides and phosphines, as described for example in EP 438123 and GB 2180358.

It is also possible to add chain transfer reagents customary in the art to the compositions of the invention. Examples are mercaptans, amines and benzothiazole.

The photopolymerization may further be accelerated by adding photosensitizers as further additives (D), which shift or broaden the spectral sensitivity. These photosensitizers are, in particular, aromatic carbonyl compounds such as benzophenone derivatives, thioxanthone derivatives, and also especially isopropylthioxanthone, anthraquinone derivatives and 3-acylcoumarin derivatives, terphenyls, styryl ketones, and also 3-(aroylmethylene)-thiazolines, camphorquinone, and also eosine dyes, rhodamine dyes and erythrosine dyes.

The amines indicated above, for example, may also be regarded as photosensitizers.

The curing process, especially of compositions which are pigmented (with titanium dioxide for example), may also be assisted by adding an additional additive (D) which is a component which under thermal conditions forms free radicals, such as an azo compound, for instance 2,2'-azobis(4-methoxy-2,4-dimethylvaleronitrile), a triazene, diazo sulfide, pentazadiene or a peroxy compound such as hydroperoxide or peroxycarbonate, e.g. t-butyl hydroperoxide, as described for example in EP 245639.

As further additives (D), the compositions may also comprise, for example, a photoreducible dye, such as xanthene, benzoxanthene, benzothioxanthene, thiazine, pyronine, porphyrin or acridine dyes, and/or a radiation-cleavable trihalomethyl compound. Similar compositions are described, for example, in EP 445624.

Further common additives (D) - depending on the intended use - include optical brighteners, fillers, e.g. kaolin, talc, barytes, gypsum, chalk or silicatic fillers, pigments, dyes, wetting agents or flow improvers. For the curing of thick and pigmented coatings it is appropriate to add glass microbeads or pulverized glass fibres, as described for example in US 5,013,768.

The formulations may also comprise dyes and/or white or coloured pigments. Depending on the intended application, both organic and inorganic pigments may be used. Such additions are known to the person skilled in the art; some examples are titanium dioxide pigments, of, for example, the rutile or anatase type, carbon black, zinc oxide, such as zinc white, iron oxides, such as yellow iron oxide, red iron oxide, chrome yellow, chrome green, nickel titanium yellow, ultramarine blue, cobalt blue, bismuth vanadate, cadmium yellow or cadmium red. Examples of organic pigments are monoazo or disazo pigments, and also metal complexes thereof, phthalocyanine pigments, polycyclic pigments, such as perylene, anthraquinone, thioindigo, quinacridone or triphenylmethane pigments, and also diketopyrrolopyrrole, isoindolinone, e.g. tetrachloroisoindolinone, isoindoline, dioxazine, benzimidazolone and quinophthalone pigments.

The pigments may be used individually or else in a mixture in the formulations.

The pigments, depending on the intended use, are added to the formulations in the amounts customary in the art, for example in an amount of from 1 to 60% by weight, or from 10 to 30% by weight, based on the overall mass.

The formulations may also, for example, comprise organic dyes from a very wide variety of classes. Examples are azo dyes, methine dyes, anthraquinone dyes or metal complex dyes. Customary concentrations are, for example, from 0.1 to 20%, in particular from 1 to 5%, based on the overall mass.

The choice of additives is guided by the respective field of application and by the properties desired for this field. The above-described additives (D) are customary in the art and, accordingly, are used in the amounts that are customary in the art.

In certain cases it may be of advantage to use mixtures of two or more of the photoinitiators of the formula I; it is advantageous, for example, to use mixtures obtained directly in the preparation. It is of course also possible to use mixtures with known photoinitiators (E), examples being mixtures with camphorquinone, benzophenone, benzophenone derivatives, acetophenone, acetophenone derivatives, such as α-hydroxycycloalkyl phenyl ketones or 2-hydroxy-2-methyl-1-phenylpropanone, dialkoxyacetophenones, α-hydroxy- or α-amino-acetophenones, such as (4-methylthiobenzoyl)-1-methyl-1-morpholinoethane, (4-morpholino-benzoyl)-1-benzyl-1-dimethylaminopropane, 4-aroyl-1,3-dioxolanes, benzoin alkyl ethers and benzil ketals, such as benzil dimethyl ketal, phenylglyoxalates and derivatives thereof, dimeric phenylglyoxalates, peresters, for example benzophenonetetracarboxylic peresters as described for example in EP 126541, monoacylphosphine oxides, such as (2,4,6-trimethylbenzoyl)phenylphosphine oxide, bisacylphosphine oxides, such as bis(2,6-dimethoxybenzoyl(2,4,4-trimethylpent-1-yl)phosphine oxide, bis(2,4,6-trimethylbenzoyl)phenylphosphine oxide or bis(2,4,6-trimethylbenzoyl)(2,4-dipentoxyphenyl)-phosphine oxide, tris-acylphosphine oxides, halomethyltriazines, e.g. 2-[2-(4-methoxyphenyl)-vinyl]-4,6-bistrichloromethyl[1,3,5]triazine, 2-(4-methoxyphenyl)-4,6-bistrichloromethyl-[1,3,5]triazine, 2-(3,4-dimethoxyphenyl)-4,6-bistrichloromethyl[1,3,5]triazine, 2-methyl-4,6-bistrichloromethyl[1,3,5]triazine, hexaarylbisimidazole / coinitiator systems, e.g. ortho-chlorohexa-phenylbisimidazole together with 2-mercaptobenzothiazole, ferrocenium compounds or titanocenes, such as dicyclopentadienylbis(2,6-difluoro-3-pyrrolophenyl)titanium or borate photoinitiators.

Where the photoinitiators of the invention are employed in hybrid systems, i.e. systems which can be cured both free-radically and cationically, use is made, in addition to the free-radical curing agents of the formula I and any further free-radical curing agents, of cationic photoinitiators such as benzoyl peroxide (other suitable peroxides are described in US 4,950,581, column 19, lines 17-25), aromatic sulfonium, phosphonium or iodonium salts, as described for example in US 4,950,581, column 18, line 60 to column 19, line 10.

The photopolymerizable compositions contain the photoinitiator appropriately in an amount of from 0.05 to 15% by weight, preferably from 0.1 to 5% by weight, based on the composition. The stated amount of photoinitiator is based on the sum of all of the added photoinitiators, if mixtures thereof are used, i.e. both on the photoinitiator (B) and on the photoinitiators (B) + (E).

The fluorinated photoinitiators are especially suitable to produce coatings for applications like glass, such as windows, shields and glass fibers; for polymers such as polyester, polyvinylchloride, polyethylene and polypropylene; polycarbonate; woven and nonwoven fabrics; paper glass and metals, such as steel and aluminum; coatings for head lamps and glass fiber coatings.

The substrates may be coated by applying a liquid composition, a solution or suspension to the substrate. The choice of solvent and the concentration are guided primarily by the nature of the composition and by the coating technique. The solvent should be inert, i.e. it should not enter into any chemical reaction with the components and it should be able to be removed again in the course of drying after coating. Examples of suitable solvents are ketones, ethers and esters, such as methyl ethyl ketone, isobutyl methyl ketone, cyclopentanone, cyclohexanone, N-methylpyrrolidone, dioxane, tetrahydrofuran, 2-methoxyethanol, 2-ethoxyethanol, 1-methoxy-2-propanol, 1,2-dimethoxyethane, ethyl acetate, n-butyl acetate and ethyl 3-ethoxypropionate.

The formulation is applied uniformly to a substrate by means of known coating techniques, for example by spincoating, dipping, knife coating, curtain coating techniques, brush application, spraying, especially by electrostatic spraying, and reverse roll coating, and also by electrophoretic deposition. It is also possible to apply the photosensitive layer to a temporary flexible support and then, by layer transfer via lamination, to the final substrate.

The application (coat thickness) and nature of the substrate (coat support) are dependent on the desired field of application. The dry film thickness range generally embraces values from about 0.1 µm to more than 100 µm, preferably from 0.02 to 2 mm.

A further field of use of photocuring is that of metal coating, as in the coating of metal sheets and tubes, cans or bottle closures, for example, and also photocuring on polymer coatings, for example PVC-based wall or floor coverings.

Examples of the photocuring of paper coatings are the colourless varnishing of labels or book covers.

The photosensitivity of the compositions of the invention generally ranges from about 200 nm to about 600 nm (UV region). Suitable radiation is present, for example, in sunlight or light from artificial sources. Light sources employed therefore include a large number of a very wide variety of types. Both point sources and arrays (lamp carpets) are suitable. Examples are carbon arc lamps, xenon arc lamps, medium-, high- and low-pressure mercury lamps, possibly doped with metal halides (metal-halogen lamps), microwave-excited metal vapour lamps, excimer lamps, superactinic fluorescent tubes, fluorescent lamps, argon incandescent lamps, flashlights, photographic floodlamps, light-emitting diodes (LEDs), electron beams and X-rays. The distance between the lamp and the substrate to be exposed may vary depending on the intended application and the type and output of the lamps, for example between 2 cm and 150 cm.

As already mentioned, curing in the process of the invention may take place solely by exposure to electromagnetic radiation. Depending on the composition of the formulation to be cured, however, thermal curing before, during or after radiation exposure is appropriate.

Thermal curing takes place in accordance with methods known to the person skilled in the art. Curing is generally carried out in an oven, e.g. a forced-air oven, on a hotplate, or by irradiation using IR lamps. Curing without auxiliaries at room temperature is likewise possible, depending on the binder system used. The curing temperatures are generally between room temperature and 150°C, e.g. 25-150°C or 50-150°C. In the case of powder coating materials or coil coating materials, the curing temperatures may also be higher, e.g. up to 350°C.

The invention additionally provides for the use of the above-described composition and to a process for producing pigmented and unpigmented paints and varnishes, powder coating materials, gel coats, composite materials or glass fibre cable coatings.

The invention likewise provides a coated substrate coated on at least one surface with a composition as described above.

The examples which follow illustrate the invention but do not indicate any intention that the invention be restricted to the examples. As in the remainder of the description and in the claims, parts and percentages are by weight unless indicated otherwise.

### Examples:

### Example 1:

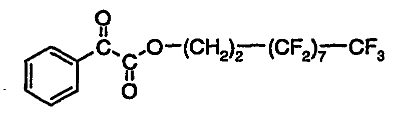

R = radical of the formula II, R₁, R₂, R₃, R₄, R₅ = H, s =1, A = -(CH₂)₂-(CF₂)₇-CF₃, Y is a bond.

A solution of 5g (10.77 mmol)1H,1H,2H,2H-perfluoro-decan-1-ol, 1.54g (10.26 mmol) benzoylformic acid(glyoxalic acid) and 0.06g (0.3 mmol) p-toluenesulfonic acid in 20ml toluene is heated at reflux for 48 hours. The mixture is poured into saturated NaHCO₃ solution and the phases are separated. The organic phases are washed with water and dried over magnesium sulfate. Filtration and evaporation of the solvent give 3.16g (52%) of orange crystals.
U.V. (CH₃CN) max. at 256 nm (ε 12 618). ¹H NMR (CDCl₃) δ [ppm]: 7.90 (m, 2 H arom.); 7.58 (m, 1 H arom.); 7.40 (m, 2 H arom.); 4.57 (t, J = 7.5, 2 H, -C(O)-O-CH₂-); 2.49 (m, 2 H, -C(O)-O-CH₂-CH₂-). m/z (Cl) 596 (M⁺).

### Example 2:

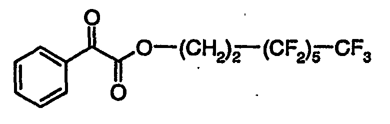

(R = radical of the formula II, R₁, R₂, R₃, R₄, R₅ = H, s = 1, Y is a bond,
A = -(CH₂)₂-(CF₂)₅-CF₃.

The compound of Example 2 is prepared by the method described in Example 1 using 1 mole equivalent of benzoylformic acid (glyoxalic acid) and 1.25 mole equivalents of 1 H,1H,2H,2H-perfluoro-octanol. Any perfluoro-octanol excess is distilled off. The residue was taken up in toluene and filtrered using Hyflo. 3.66g (67%) of a yellow oil is obtained.
U.V. (CH₃CN) max. bei 255 nm (ε 12'090). ¹H-NMR (CDCl₃) δ [ppm ]: 7.91 (m, 2 H arom.); 7.57 (m, 1 H arom.); 7.42 (m, 2 H arom.); 4.58 (t, J = 6.6, 2 H, -C(O)-O-CH₂-); 2.54 (m, 2 H, -C(O)-O-CH₂-CH₂-). m/z (El + Cl) 497 (MH⁺); According to the mass spectrum there is also a further compound present in a small amount. 597 (MH⁺); 525 (MH⁺).

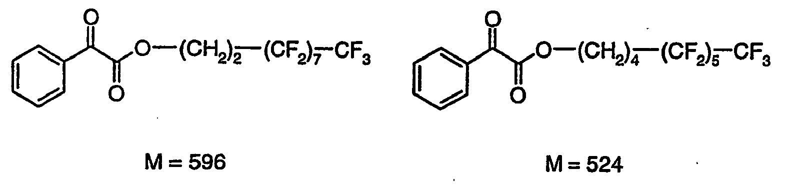

### Example 3:

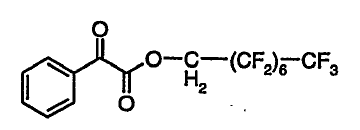

(R = radical of the formula II, R₁, R₂, R₃, R₄, R₅ = H, s = 1, Y is a bond,
A = -CH₂-(CF₂)₆-CF₃.

The compound of Example 3 is prepared by the method described in Example 1 using 1 mole equivalent of benzoylformic acid (glyoxalic acid) and 1.25 mole equivalents of 1H,1H,-perfluoro-octanol. 0.78g (37%) of a yellow oil is obtained.
U.V. (CH₃CN) max. bei 256 nm (ε 4'699). ¹H-NMR (CDCl₃) δ [ppm ]: 7.89 (m; 2 H arom.); 7.58 (m, 1 H arom.); 7.42 (m, 2 H arom.); 4.78 (AB Syst, 2 H, -C(O)-O-CH₂-). m/z (El + Cl) 533 (MH⁺); According to the mass spectrum there is also a further compound present in a small amount: 519 (MH⁺); 483 (MH⁺); 400 (M⁺).

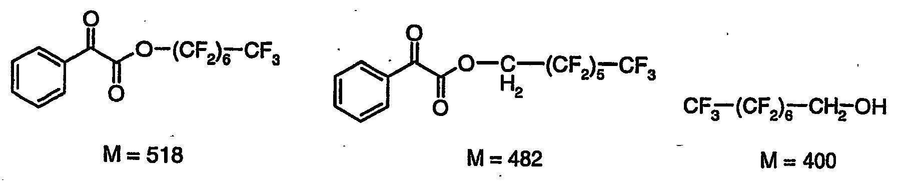

### Example 4:

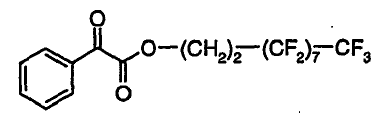

(R = radical of the formula II, R₁, R₂, R₃, R₄, R₅ = H, s = 1, Y is a bond ,
A = -(CH₂)₂-(CF₂)₇-CF₃.

The compound of Example 4 is prepared by the method described in Example 1 using 1 mole equivalent of benzoylformic acid (glyoxalic acid) and 1.25 mole equivalents of 1 H,1H,2H,2H-perfluoro-decanol. 4.08g (79%) of a yellow solid is obtained.
U.V. (CH₃CN) max. bei 255 nm (ε 11'980). ¹H-NMR (CDCl₃) δ [ppm ]: 7.94 (m, 2 H arom.); 7.61 (m, 1 H arom.); 7.45 (m, 2 H arom.); 4.78 (t, J = 6.7, 2 H, -C(O)-O-CH₂-); 2.56 (m, 2 H, -C(O)-O-CH₂-CH₂-). m/z (El + Cl) 597 (MH⁺); According to the mass spectrum there is also a further compound present in a small amount: 583 (MH⁺); 497 (MH⁺); 465 (MH⁺).

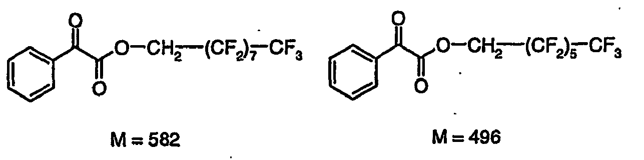

### Example 5:

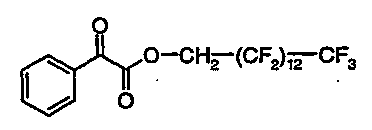

(R = radical of the formula II, R₁, R₂, R₃, R₄, R₅ = H, s = 1, Y is a bond, A = -CH₂-(CF₂)₁₂-CF₃. The compound of Example 5 is prepared by the method described in Example 1 using 1 mole equivalent of benzoylformic acid (glyoxalic acid) and 1.25 mole equivalents of 1 H,1H,-perfluoro-1-tetradecanol. 1.96g (68%) of a white crystals is obtained. mp = 151 °C. U.V. (CH₃CN) max. bei 241 nm (ε 3'359). ¹H-NMR (CDCl₃) δ [ppm ]: 8.01 (m, 2 H arom.); 7.72 (m, 1 H arom.); 7.54 (m, 2 H arom.); 4.90 (AB Syst, 2 H, -C(O)-O-CH₂-); 2.56 (m, 2 H, -C(O)-O-CH₂-CH₂-). m/z (El + Cl) 833 (MH⁺); According to the mass spectrum there is also a further compound present in a small amount: 701 (MH⁺).

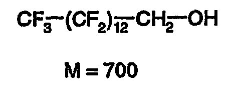

### Example 6

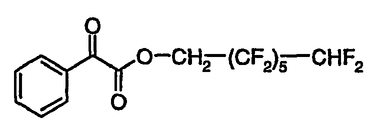

(R = radical of the formula II, R₁, R₂, R₃, R₄, R₅ = H, s = 1, Y is a bond, A = -CH₂-(CF₂)₅-CHF₂

The compound of Example 6 is prepared by the method described in Example 1 using 1 mole equivalent of benzoylformic acid (glyoxalic acid) and 1.25 mole equivalents of 1 H,1H,-7H-perfluoro-1-heptanol. Any perfluoro-heptanol excess is distilled off. The residue was taken up in toluene containing activated carbon and filtrered using Hyflo. 6.72g (60%) of a yellow oil is obtained.
U.V. (CH₃CN) max. bei 258 nm (ε 5'813). ¹H-NMR (CDCl₃) δ [[ppm ]: 7.91 (m, 2 H arom.); 7.70 (m, 1 H arom.); 7.54 (m, 2 H arom.); 5.96 (t x t, J = 5.1, 51.5, 1H, -CHF₂); 4.90 (t, J = 13.5, 2 H, -C(O)-O-CH₂-). m/z (El + Cl) 465 (MH⁺).

### Examples 7

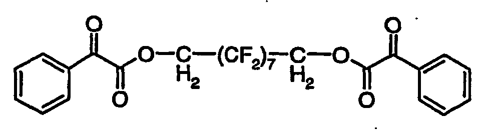

and 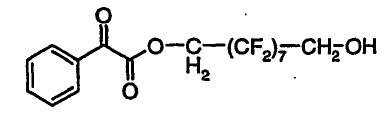 (Diester: R = radical of the formula II, R₁, R₂, R₃, R₄, R₅ = H, s = 2, Y is a bond, A = -CH₂-(CF₂)₇-CH₂-.
(Monoester: R = radical of the formula II, R₁, R₂, R₃, R₄, R₅ = H, s =1, Y is a bond, A = -CH₂-(CF₂)₇-CH₂-OH.

A solution of 3g (10 mmol) (dimethylamino)phenyl-diphenylphosphine in THF (83 ml) is cooled at -10°C and 1.99 g (10 mmol) azodicarboxylic acid-diisopropylester is added. 2.03 g (5 mmol)1H,1H,9H,9H-perfluoro-1-9-nonadiol and 1.48 g (10 mmol) benzoylformic acid in THF (17 ml) are added to this mixture. The solution is allowed to warm to room temperature and stirred for 26 hours. The reaction mixture is evaporated. The residue is dissolved in diethyl ether, and successively washed with 5% HCl, saturated aqueous sodium hydrogencarbonate, and NaCl solution. The organic phases are dried over magnesium sulfate. Filtration, evaporation of the solvent and chromatography (Hexan/Etaylacetat 3:1) give (0.3 g, 9%) of the diester and (0.48 g, 17%) of the monoester.

Diester: ¹H-NMR (CDCl₃) δ [ppm ]: 8.00 (m, 2 x 2 H arom.); 7.70 (m, 2 x 1 H arom.); 7.52 (m, 2 x 2 H arom.); 4.87 (AB Syst., 2 x 2 H, 2 -C(O)-O-CH₂-).m/z (El + Cl) 677 (MH⁺); According to the mass spectrum there is also a further compound present in a small amount: 627 (MH⁺). 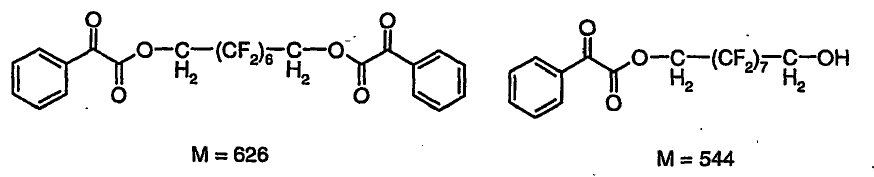 Monoester: ¹H-NMR (CDCl₃) δ [ppm]: 7.94 (m, 2 H arom.); 7.63 (m, 1 H arom.); 7.47 (m, 2 H arom.); 4.81 (t, J = 13.5, 2 H, -C(O)-O-CH₂-); 4.02 (t, J =13.8, 2 H, -(CF₂)₇-CH₂-OH). m/z (El + Cl) 676 (MH⁺); According to the mass spectrum there is also a further compound present in a small amount: 494 (MH⁺).

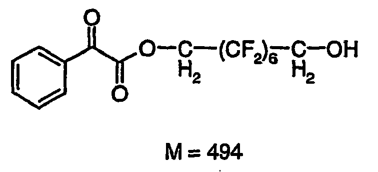

### Example 8:

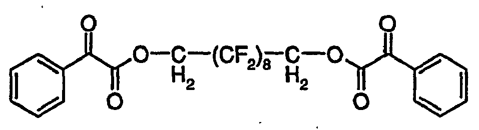

(R = radical of the formula II, R₁, R₂, R₃, R₄, R₅ = H, s = 2, Y is a bond, A = -CH₂-(CF₂)₈-CH₂-

The compound of Example 8 is prepared by the method described in Example 1 using 2 mole equivalent of benzoylformic acid (glyoxalic acid) and 1.25 mole equivalents of 1H,1H,2H,2H-perfluoro-decanol. After chromatography (hexanelethylacetate 1:1) (0.31 g, 10 %) of the diester and (0.82g, 32%) of the monoester is obtained both as white solids. Diester: ¹H-NMR (CDCl₃) δ [ppm ]: 8.10 (m, 2 x 2 H arom.); 7.75 (m, 2 x 1H arom.); 7.67 (m, 2 x 2 H arom.); 5.00 (t, J = 13.5, 2 x 2 H, 2 -C(O)-O-CH₂-). m/z (El + Cl) 727 Monoester: ¹H-NMR (CDCl₃) δ [ppm ]: 8.00 (m, 2 H arom.); 7.71 (m, 1 H arom.); 7.49 (m, 2 H arom.); 4.88 (t, J =13.5, 2 H, -C(O)-O-CH₂-); 4.88 (t, J = 13.5, 2 H, -(CF₂)₈-CH₂-OH). m/z (El + Cl) 595 (MH⁺); According to the mass spectrum there is also a further compound present in a small amount: 727 (MH⁺).

### Beispiel 9:

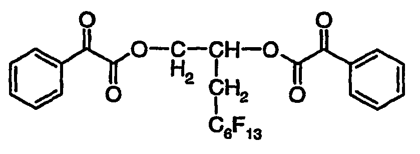

(R = radical of the formula II, R₁, R₂, R₃, R₄, R₅ = H, s = 2, Y is a bond,
A = -CH₂-CH(CH₂-C₆F₁₃)-

The compound of Example 9 is prepared by the method described in Example 1 using 1.6 mole equivalent of benzoylformic acid (glyoxalic acid) and 1. mole equivalents of 1 H,1H,-2H,3H,3H-perfluoro-nonan-1,2-diol. After chromatography (hexane/ethylacetate 3:1) (1.26g, 8 %) of a colorless oil is obtained.
¹H-NMR (CDCl₃) δ [ppm ]: 8.11 (m, 2 x 1 H arom.); 7.94 (m, 2 x 1 H arom.); 7.78 (m, 2 x 1H arom.); 7.58 (m, 2 x 2 H arom.); 5.02 (m, 1 H, -CH-CH₂-C₆F₁₃); 4.96 (m, 1 H, -CH₂-O-COCO-Ph); 4.66 (m, 1 H, -CH₂-O-CO-CO-Ph); 3.16-2.75 (m, 2 H, <-CH₂-C₆F₁₃). m/z (El + Cl) 659 (MH⁺).

### Example 10

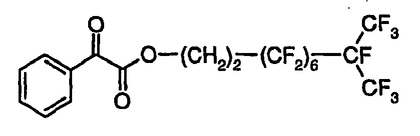

(R = radical of the formula II, R₁, R₂, R₃, R₄, R₅ = H, s = 1, Y is a bond,
A = -(CH₂)₂-(CF₂)₆-CF(CF₃)₂)

The compound of Example 10 is prepared by the method described in Example 1 using 1 mole equivalent of benzoylformic acid (glyoxalic acid) and 1.25 mole equivalents of 1H,1H,2H,2H-perfluoro-9-methyldecane-1-ol. After chromatography (hexane/ethylacetate 3:1) a colorless oil is obtained.
U.V. (CH₃CN) max. bei 258 nm (ε 5'813). ¹H-NMR (CDCl₃) δ [ppm ]: 7.91 (m, 2 H arom.); 7.70 (m, 1 H arom.); 7.54 (m, 2 H arom.); 5.96 (t x t, J = 5.1, 51.5, 1 H, -CHF₂); 4.90 (t, J = 13.5, 2 H, -C(O)-O-CH₂-). m/z (El + Cl) 465 (MH⁺).

### Application example:

A formulation containing 1H,1H-Perfluoro-n-oktyl acrylate (C8ACRY from Exfluor) and 3% of the compound of Example 1 is applied on a glass plate with a dry film thickness of 10µm.

The formulation is cured with 2X120 W/cm mercury medium pressure lamps at a belt speed of 5 m/min. A tack free cured film is obtained.

## Claims

1. A photopolymerizable composition comprising
(A) at least one ethylenically unsaturated photopolymerizable fluocarbon compound selected from polyfluoroalkyl monoacrylates, polyfluoroalkyl monomethacrylates or polyfluoroalkyldiacrylates and
(B) at least one photoinitiator of the formula I 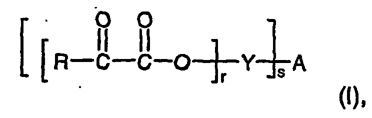
in which
**R** is a radical of the formula II 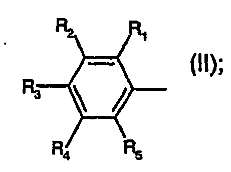 or
**R** is naphthyl, anthracyl, phenanthryl or a heterocyclic radical,
the radicals naphthyl, anthracyl, phenanthryl and/or the heterocycle being unsubstituted or substituted by C₁-C₈alkyl, phenyl, OR₆, SR₇ and/or NR₈R₉, where the substituents OR₆, SR₇, NR₈R₉ may form 5- or 6-membered rings via the radicals R₆, R₇, R₈ and/or R₉ with further substituents on the naphthyl, anthracyl or phenanthryl ring or on the heterocycle or with one of the carbon atoms of the naphthyl, anthracyl or phenanthryl ring or with one of the carbon atoms of the heterocycle;
and
R₁, R₂, R₃, R₄ and R₅ independently of one another are hydrogen; unsubstituted C₁-C₁₂alkyl or C₁-C₁₂alkyl substituted by OH, C₁-C₄alkoxy, phenyl, naphthyl, halogen, CN and/or -O(CO)R₁₀; or are C₂-C₁₂alkyl interrupted by one or more non-successive oxygen atoms; or are OR₆; SR₇; NR₈R₉; halogen; unsubstituted or C₁-C₄alkyl- and/or C₁-C₄alkoxy-substituted phenyl, where the substituents OR₆, SR₇, NR₈R₉ may form 5- or 6-membered rings via the radicals R₆, R₇, R₈ and/or R₉ with further substituents on the phenyl ring or one of the carbon atoms of the phenyl ring;
R₆ and R₇ independently of one another are hydrogen; unsubstituted C₁-C₁₂alkyl or C₁-C₁₂alkyl substituted by OH, C₁-C₄alkoxy, phenyl, phenoxy and/or -O(CO)R₁₀; or are C₂-C₁₂alkyl interrupted by one or more non-successive oxygen atoms; or are unsubstituted phenyl, C₃-C₆alkenyl, cyclopentyl, cyclohexyl or naphthyl; or are C₁-C₄alkoxy-, phenyl- and/or C₁-C₄alkyl-substituted phenyl, C₃-C₆alkenyl, cyclopentyl, cyclohexyl or naphthyl;
R₈ and R₉ independently of one another are hydrogen; unsubstituted C₁-C₁₂alkyl or C₁-C₁₂alkyl substituted by OH, C₁-C₄alkoxy and/or phenyl; or are C₂-C₁₂alkyl interrupted by one or more non-successive oxygen atoms; or are phenyl, -(CO)R₁₀ or SO₂R₁₁; or
R₈ and R₉, together with the nitrogen atom to which they are attached, form a 5-, 6- or 7-membered ring which is uninterrupted or interrupted by -O- or -NR₁₂-;
R₁₀ is C₁-C₈alkyl; unsubstituted phenyl; or phenyl substituted by C₁-C₄alkyl and/or C₁-C₄alkoxy;
R₁₁ is C₁-C₁₂alkg, unsubstituted phenyl or phenyl substituted by C₁-C₄alkyl;
R₁₂ is hydrogen; unsubstituted C₁-C₈alkyl; C₁-C₈alkyl substituted by OH or C₁-C₄alkoxy; unsubstituted phenyl; phenyl substituted by OH, C₁-C₄alkyl and/or C₁-C₄alkoxy; and
s is a number of 1 to 4;
r is a number of 1 or 2;
Y is a single bond or is a divalent group C₁-C₁₀alkylene, C₂-C₁₀alkenylene, C₃-C₁₀alkynylene, -(CH₂)ₐ-O-, -[(CH₂)ₐ-O-(CH₂)_{b}]_{c}-, -[(CH₂)ₐ-O]_{q}-(CH₂)_{b}-, -(CH₂)ₐ-O-(CH₂)_{b}-O-, -(CH₂)ₐ-NR₈-(CH₂)_{b}-, -(CH₂)ₐ-O-(CH₂)_{b}-NR₈-(CH₂)_{c}-, -(C₂-C₁₀alkenylene)-O-(CH₂)ₐ-, -(C₂-C₁₀alkynylene)-O-(CH₂)ₐ-, with the proviso that at the site of a bond to the photoinitiator radical 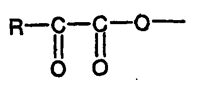 there must always be at least one methylene group;
a is a number from 1 to 10;
b and c independently of one another are a number from 0 to 10; with the proviso that they are, however, at least 1 if the methylene group in question is between two oxygen atoms or one oxygen and one nitrogen atom, and
q is a number from 1 to 30.
**A** **if s is 1,** is a radical A₀, where A₀ is C₁-C₃₀alkyl, C₁-C₃₀alkenyl, C₁-C₃₀alkynyl or C₁-C₃₀aralkyl, **the radicals being substituted by at least two fluorine** atoms and optional further substituted by OH-, C₁-C₄alkoxy-, phenyl-, naphthyl-, halogen-, CN-, SRr, NR₈R₉- and/or -O(CO)R₁₀-; and the radical A₀ is uninterrupted or interrupted by one or more -O-, -S- or -NR₁₂-;
**A** **if s is >1,** is a radical A₁; where A₁ is C₁-C₃₀alkylene, C₁-C₃₀alkenylene, C₁-C₃₀alkynylene or C₁-C₃₀aralkylene, **the radicals being substituted by at least two fluorine** atoms and optionally further substituted by OH-, C₁-C₄alkoxy-, phenyl-, naphthyl-, halogen-, CN-, SR₇-, NR₈R₉- and/or -O(CO)R₁₀-; and the radical A₁ is uninterrupted or interrupted by one or more -O-, -S- or -NR₁₂-.

2. A photopolymerizable composition according to claim 1, wherein
**R** is a radical of the formula II in which R₁, R₂, R₃, R₄ and R₅ independently of one another are hydrogen or C₁-C₄alkyl or C₁-C₄alkoxy;
**Y** is a single bond,
**A** **if s is 1,** is a radical A₀, A₀ being C₆-C₃₀alkyl substituted by seven or more fluorine atoms and optionally further substituted by OH.
**A** **if s is >1,** is a radical A₁; A₁ being C₆-C₃₀alkylene substituted by seven or more fluorine atoms and optionally further substituted by OH.

3. A photopolymerizable composition according to claim 1, wherein
**R** is phenyl,
**Y** is a single bond;
**A** **if s is 1,** is a radical A₀, A₀ being -(CH₂)₂-(CF₂)₅-CF₃,-(CH₂)₂-(CF₂)₆-CF(CF₃)₂, -CH₂-(CF₂)₆-CF₃, -(CH₂)₂-(CF₂)₇-CF₃, -CH₂-(CF₂)₁₂-CF₃, -CH₂-(CF₂)₅-CHF₂, -CH₂-(CF₂)₇-CH₂-OH
**A** **if s is >1,** is a radical A₁;A₁ being : -CH₂-(CF₂)₇-CH₂-, -CH₂-(CF₂)₈-CH₂-, -CH₂-CH(CH₂-C₆F₁₃)-.

4. A composition according to any one of claims 1 -3 comprising in addition to components (A) and (B) at least one thermally crosslinkable compound (C).

5. A composition according to claim 4, comprising in addition to components (A) and (B), or (A), (B) and (C), further additives (D) and/or additional photoinitiators (E).

6. A process for producing coatings having stable scratch-resistant surfaces, in which
(1) a photocurable composition according to any one of calims 1-3 is prepared;
(2) this composition is applied to a substrate; and
(3) the composition is cured either
only by exposure to electromagnetic radiation with a wavelength ranging from 200 nm into the IR region, or
by exposure to electromagnetic radiation with a wavelength ranging from 200 nm into the IR region and prior, simultaneous and/or subsequent exposure to heat.

7. The use of compositions according to any one of claims 1-3 for applications like glass, such as windows, shields and glass fibers; for polymers such as polyester, polyvinylchloride, polyethylene and polypropylene; polycarbonate; woven and nonwoven fabrics; paper glass and metals, such as steel and aluminum; coatings for head lamps and glass fiber coatings.

## Patentansprüche

1. Photopolymerisierbare Zusammensetzung, umfassend
(A) mindestens eine ethylenisch ungesättigte, photopolymerisierbare Fluorkohlenstoff-Verbindung, ausgewählt aus Polyfluoralkylmonoacrylaten, Polyfluoralkylmonomethacrylaten oder Polyfluoralkyldiacrylaten, und
(B) mindestens einen Photostarter der Formel I 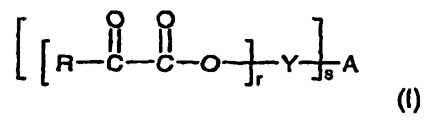
worin
**R** einen Rest der Formel II 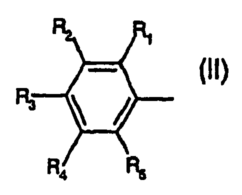 darstellt; oder
**R** Naphthyl-, Anthracyl-, Phenanthryl- oder einen heterocyclischen Rest darstellt, wobei die Reste Naphthyl, Anthracyl, Phenanthryl und/oder der Heterocyclus unsubstituiert oder mit C₁-C₈-Alkyl, Phenyl, OR₆, SR₇ und/oder NR₈R₉ substituiert sind, wobei die Substituenten OR₆, SR₇, NR₈R₉ 5- oder 6-gliedrige Ringe über die Reste R₆, R₇, R₈ und/oder R₉ mit weiteren Substituenten an dem Naphthyl-, Anthracyl- oder Phenanthrylring oder an dem Heterocyclus oder mit einem der Kohlenstoffatome von dem Naphthyl-, Anthracyl- oder Phenanthrylring oder mit einem der Kohlenstoffatome von dem Heterocyclus bilden können; und
R₁, R₂, R₃, R₄ und R₅ unabhängig voneinander Wasserstoff; unsubstituiertes C₁-C₁₂-Alkyl, oder C₁-C₁₂-Alkyl, substituiert mit OH, C₁-C₄-Alkoxy, Phenyl, Naphthyl, Halogen, CN und/oder -O(CO)R₁₀ darstellen; oder C₂-C₁₂-Alkyl, unterbrochen durch ein oder mehrere nicht aufeinander folgende Sauerstoffatome, darstellen; oder OR₆; SR₇; NR₈R₉; Halogen; unsubstituiertes oder C₁-C₄-Alkyl- und/oder C₁-C₄-Alkoxy-substituiertes Phenyl darstellen, wobei die Substituenten OR₆, SR₇, NR₈R₉ 5- oder 6-gliedrige Ringe über die Reste R₆, R₇, R₈ und/oder R₉ mit weiteren Substituenten an dem Phenylring oder einem der Kohlenstoffatome des Phenylrings bilden können;
R₆ und R₇ unabhängig voneinander Wasserstoff; unsubstituiertes C₁-C₁₂-Alkyl, oder C₁-C₁₂-Alkyl, substituiert mit OH, C₁-C₄-Alkoxy, Phenyl, Phenoxy und/oder -O(CO)R₁₀, darstellen; oder C₂-C₁₂-Alkyl, unterbrochen durch ein oder mehrere nicht aufeinander folgende Sauerstoffatome, darstellen; oder unsubstituiertes Phenyl, C₃-C₆-Alkenyl, Cyclopentyl, Cyclohexyl oder Naphthyl darstellen; oder C₁-C₄-Alkoxy-, Phenyl- und/oder C₁-C₄-Alkylsubstituiertes Phenyl, C₃-C₆-Alkenyl, Cyclopentyl, Cyclohexyl oder Naphthyl darstellen;
R₈ und R₉ unabhängig voneinander Wasserstoff; unsubstituiertes C₁-C₁₂-Alkyl, oder C₁-C₁₂-Alkyl, substituiert mit OH, C₁-C₄-Alkoxy und/oder Phenyl, darstellen; oder C₂-C₁₂-Alkyl, unterbrochen durch ein oder mehrere nicht aufeinander folgende Sauerstoffatome, darstellen; oder Phenyl, -(CO)R₁₀ oder SO₂R₁₁ darstellen; oder
R₈ und R₉, zusammen mit dem Stickstoffatom, an das sie gebunden sind, einen 5-, 6- oder 7-gliedrigen Ring bilden, der nicht unterbrochen oder durch -O- oder -NR₁₂- unterbrochen ist;
R₁₀ C₁-C₈-Alkyl; unsubstituiertes Phenyl; oder Phenyl, substituiert mit C₁-C₄-Alkyl und/oder C₁-C₄-Alkoxy, darstellt;
R₁₁ C₁-C₁₂-Alkyl, unsubstituiertes Phenyl oder Phenyl, substituiert mit C₁-C₄-Alkyl, darstellt;
R₁₂ Wasserstoff; unsubstituiertes C₁-C₈-Alkyl; C₁-C₈-Alkyl, substituiert mit OH oder C₁-C₄-Alkoxy; unsubstituiertes Phenyl; Phenyl, substituiert mit OH, C₁-C₄-Alkyl und/oder C₁-C₄-Alkoxy, darstellt; und
s eine Zahl von 1 bis 4 ist;
r eine Zahl von 1 oder 2 ist;
Y eine Einfachbindung oder eine zweiwertige Gruppe C₁-C₁₀-Alkylen, C₂-C₁₀-Alkenylen, C₃-C₁₀-Alkinylen, -(CH₂)ₐ-O-, -[(CH₂)ₐ-O-(CH₂)_{b}]_{c}-, -[(CH₂)ₐ-O]_{q}-(CH₂)_{b}-, -(CH₂)ₐ-O-(CH₂)_{b}-O-, -(CH₂)ₐ-NR₈-(CH₂)_{b}-, -(CH₂)ₐ-O-(CH₂)_{b}-NR₈-(CH₂)_{c}-, -(C₂-C₁₀-Alkenylen)-O-(CH₂)ₐ-, -(C₂-C₁₀-Alkinylen)-O-(CH₂)ₐ- darstellt, mit der Maßgabe, dass an einer Seite einer Bindung an dem Photostarterrest 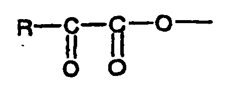
es immer mindestens eine Methylengruppe geben muss;
a eine Zahl von 1 bis 10 ist;
b und c unabhängig voneinander eine Zahl von 0 bis 10 sind; mit der Maßgabe, dass sie jedoch mindestens 1 sind, wenn die infrage kommende Methylengruppe zwischen zwei Sauerstoffatomen oder einem Sauerstoff- und einem Stickstoffatom vorliegt, und
q eine Zahl von 1 bis 30 ist.
**A,** **wenn s 1 ist,** einen Rest A₀ darstellt, wobei A₀ C₁-C₃₀-Alkyl, C₁-C₃₀-Alkenyl, C₁-C₃₀-Alkinyl oder C₁-C₃₀-Aralkyl darstellt, wobei die Reste mit mindestens zwei Fluora**tomen substituiert sind,** und gegebenenfalls weiter substituiert sind mit OH-, C₁-C₄-Alkoxy-, Phenyl-, Naphthyl-, Halogen-, CN-, SR₇-, NR₈R₉- und/oder -O(CO)R₁₀-; und der Rest A₀ nicht unterbrochen oder durch ein oder mehrere -O-, -S- oder -NR₁₂- unterbrochen ist;
**A,** **wenn s > 1,** einen Rest A₁ darstellt; worin A₁ C₁-C₃₀-Alkylen, C₁-C₃₀-Alkenylen, C₁-C₃₀-Alkinylen oder C₁-C₃₀-Aralkylen darstellt, wobei **die Reste mit mindestens zwei Fluoratomen substituiert** sind und gegebenenfalls weiter substituiert sind mit OH-, C₁-C₄-Alkoxy-, Phenyl-, Naphthyl-, Halogen-, CN-, SR₇-, NR₈R₉- und/oder -O(CO)R₁₀-; und der Rest A₁ nicht unterbrochen oder durch ein oder mehrere -O-, -S- oder -NR₁₂- unterbrochen ist.

2. Photopolymerisierbare Zusammensetzung nach Anspruch 1, worin
**R** einen Rest der Formel II darstellt, worin R₁, R₂, R₃, R₄ und R₅ unabhängig voneinander Wasserstoff oder C₁-C₄-Alkyl oder C₁-C₄-Alkoxy darstellen;
**Y** eine Einfachbindung darstellt,
**A,** **wenn s 1 ist,** einen Rest A₀ darstellt, A₀ C₆-C₃₀-Alkyl, substituiert mit sieben oder mehr Fluoratomen, und gegebenenfalls weiter substituiert mit OH, darstellt,
**A,** **wenn s > 1 ist,** einen Rest A₁ darstellt; wobei A₁ C₆-C₃₀-Alkylen, substituiert mit sieben oder mehr Fluoratomen, und gegebenenfalls weiter substituiert mit OH, darstellt.

3. Photopolymerisierbare Zusammensetzung nach Anspruch 1, worin
**R** Phenyl darstellt,
**Y** eine Einfachbindung darstellt;
**A,** **wenn s 1 ist,** einen Rest A₀ darstellt, wobei A₀ -(CH₂)₂-(CF₂)₅-CF₃, -(CH₂)₂-(CF₂)₆-CF(CF₃)₂, -CH₂-(CF₂)₆-CF₃, -(CH₂)₂-(CF₂)₇-CF₃, -CH₂-(CF₂)₁₂-CF₃, -CH₂-(CF₂)₅-CHF₂, -CH₂-(CF₂)₇-CH₂-OH dartellt.
**A,** **wenn s > 1 ist,** einen Rest A₁ darstellt; wobei A₁ bedeutet: -CH₂- (CF₂)₇-CH₂-, -CH₂- (CF₂)₈-CH₂-, -CH₂-CH (CH₂-C₆F₁₃)-.

4. Zusammensetzung nach einem der Ansprüche 1 bis 3, umfassend zusätzlich zu Komponenten (A) und (B) mindestens eine thermisch vernetzbare Verbindung (C).

5. Zusammensetzung nach Anspruch 4, umfassend zusätzlich zu Komponenten (A) und (B) oder (A), (B) und (C) weitere Additive (D) und/oder zusätzliche Photostarter (E).

6. Verfahren zur Herstellung von Beschichtungen mit stabilen, kratzbeständigen Oberflächen, wobei
(1) eine photohärtbare Zusammensetzung nach einem der Ansprüche 1-3 hergestellt wird;
(2) diese Zusammensetzung auf ein Substrat aufgetragen wird; und
(3) die Zusammensetzung gehärtet wird, entweder
nur durch Aussetzen von elektromagnetischer Strahlung mit einer Wellenlänge im Bereich von 200 nm bis in den IR-Bereich, oder
durch Aussetzen von elektromagnetischer Strahlung mit einer Wellenlänge im Bereich von 200 nm bis in den IR-Bereich, und vorher, gleichzeitig und/oder anschließend Aussetzen von Wärme.

7. Verwendung von Zusammensetzungen nach einem der Ansprüche 1-3 für Anwendungen, wie Glas, zum Beispiel Fenster, Abschirmungen und Glasfasern; für Polymere, wie Polyester, Polyvinylchlorid, Polyethylen und Polypropylen; Polycarbonat; gewebte und Vliestextilien; Papier, Glas und Metalle, wie Stahl und Aluminium; Beschichtungen für Scheinwerfer und Glasfaserbeschichtungen.

## Revendications

1. Composition photopolymérisable comprenant :
(A) au moins un composé fluorocarboné photopolymérisable éthyléniquement insaturé choisi parmi les poly(monoacrylate de fluoroalkyle), les poly(monométhacrylate de fluoroalkyle) ou les poly(diacrylate de fluoroalkyle) et
(B) au moins un photoamorceur de formule I : 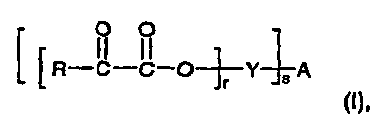
dans laquelle
R représente un radical de formule II 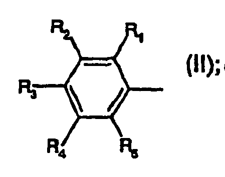 ou
R représente un groupe naphtyle, anthracyle, phénanthryle ou un radical hétérocyclique,
les groupes naphtyle, anthracyle, phénanthryle et/ou le radical hétérocyclique non substitué ou substitué par un groupe alkyle en C₁ à C₈, phényle, OR₆, SR₇ et/ou NR₈R₉, où les substituants OR₆, SR₇, NR₈R₉ peuvent former des cycles de 5 ou 6 éléments par l'intermédiaire des radicaux R₆, R₇, R₈ et/ou R₉ avec d'autres substituants sur le cycle naphtyle, anthracyle ou phénanthryle ou sur l'hétérocycle ou avec un des atomes de carbone du cycle naphtyle, anthracyle ou phénanthryle ou avec l'un des atomes de carbone de l'hétérocycle ;
et
R₁, R₂, R₃, R₄ et R₅, indépendamment les uns des autres, représentent un atome d'hydrogène ; un groupe alkyle en C₁ à C₁₂ non substitué ou un groupe alkyle en C₁ à C₁₂ substitué par un groupe OH, alcoxy en C₁ à C₄, phényle, naphtyle, halogéno, CN et/ou -O(CO)R₁₀ ; ou bien ils représentent un groupe alkyle en C₂ à C₁₂ interrompu par un ou plusieurs atomes d'oxygène non successifs ; ou bien ils représentent OR₆ ; SR₇ ; NR₈R₉ ; un atome d'halogène ; un groupe phényle non substitué ou substitué par un groupe alkyle en C₁ à C₄ et/ou alcoxy en C₁ à C₄, où les substituants OR₆, SR₇, NR₈R₉ peuvent former des cycles de 5 ou 6 éléments par l'intermédiaire des radicaux R₆, R₇, R₈ et/ou R₉ avec d'autres substituants sur le cycle phényle ou un des atomes de carbone du cycle phényle ;
R₆ et R₇ représentent, indépendamment l'un de l'autre, un atome d'hydrogène ; un groupe alkyle en C₁ à C₁₂ non substitué ou un groupe alkyle en C₁ à C₁₂ substitué par un groupe OH, alcoxy en C₁ à C₄, phényle, phénoxy et/ou -O(CO)R₁₀ ; ou bien ils représentent un groupe alkyle en C₂ à C₁₂ interrompu par un ou plusieurs atomes d'oxygène non successifs ; ou bien ils représentent un groupe phényle non substitué, alcényle en C₃ à C₆, cyclopentyle, cyclohexyle ou naphtyle ; ou bien ils représentent un groupe alcoxy en C₁ à C₄, phényle et/ou phényle substitué par un groupe alkyle en C₁ à C₄, alcényle en C₃ à C₆, cyclopentyle, cyclohexyle ou naphtyle ;
R₈ et R₉ représentent, indépendamment les uns des autres, un atome d'hydrogène ; un groupe alkyle en C₁ à C₁₂ non substitué ou alkyle en C₁ à C₁₂ substitué par un groupe OH, alcoxy en C₁ à C₄ et/ou phényle ; ou bien ils représentent un groupe alkyle en C₂ à C₁₂ interrompu par un ou plusieurs atomes d'oxygène non successifs ; ou bien ils représentent un groupe phényle, -(CO)R₁₀ ou SO₂R₁₁ ; ou
R₈ et R₉, conjointement avec l'atome d'azote auquel ils sont liés, forment un cycle de 5, 6 ou 7 éléments qui est ininterrompu ou interrompu par -O- ou -NR₁₂- ;
R₁₀ représente un groupe alkyle en C₁ à C₈ ; un groupe phényle non substitué ; ou un groupe phényle substitué par un groupe alkyle en C₁ à C₄ et/ou alcoxy en C₁ à C₄ ;
R₁₁ représente un groupe alkyle en C₁ à C₁₂, un groupe phényle non substitué ou substitué par un groupe alkyle en C₁ à C₄ ;
R₁₂ représente un atome d'hydrogène ; un groupe alkyle en C₁ à C₈ non substitué ; un groupe alkyle en C₁ à C₈ substitué par un groupe OH ou alcoxy en C₁ à C₄ ; un groupe phényle non substitué ; un groupe phényle substitué par OH, alkyle en C₁ à C₄ et/ou alcoxy en C₁ à C₄ ; et
s représente un nombre allant de 1 à 4 ;
r représente un nombre valant 1 ou 2 ;
Y est une simple liaison ou représente un groupe divalent, alkylène en C₁ à C₁₀, alcénylène en C₂ à C₁₀, alcynylène en C₃ à C₁₀, -(CH₂)ₐ-O-, - [(CH₂)ₐ-O-(CH₂)_{b}]_{c}-, - [(CH₂)ₐ-O]_{q}- (CH₂)_{b}-, -(CH₂)ₐ-O-(CH₂)_{b}-O-, -(CH₂)ₐ-NR₈-(CH₂)_{b}-, (CH₂)ₐ-O-(CH₂)_{b}-NR₈-(CH₂)_{c}-, -(alcénylène en C₂ à C₁₀)-O-(CH₂)ₐ-, -(alcynylène en C₂ à C₁₀)-O-(CH₂)ₐ-, à condition que, au niveau du site d'une liaison au photoamorceur radicalaire 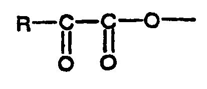 il y ait toujours au moins un groupe méthylène ;
a est un nombre allant de 1 à 10 ;
b et c, indépendamment l'un de l'autre, représentent un nombre allant de 0 à 10 ; à condition qu'ils valent, toutefois, au moins 1 si le groupe méthylène en question est entre deux atomes d'oxygène ou un atome d'oxygène et un atome d'azote, et
q est un nombre compris entre 1 et 30,
A si s vaut 1, représente un radical A₀ ; où A₀ représente un groupe alkyle en C₁ à C₃₀, alcényle en C₁ à C₃₀, alkynyle en C₁ à C₃₀ ou aralkyle en C₁ à C₃₀, les radicaux étant substitués par au moins deux atomes de fluor et éventuellement en outre substitués par un groupe OH, alcoxy en C₁ à C₄, phényle, naphtyle, halogéno, CN-, SR₇-, NR₈R₉- et/ou -O(CO)R₁₀- ; et le radical A₀ est ininterrompu ou interrompu par un ou plusieurs -O-, -S- ou -NR₁₂- ;
A si s est supérieur à 1, représente un radical A₁ ; où A₁ représente un groupe alkylène en C₁ à C₃₀, alcénylène en C₁ à C₃₀, alcynylène en C₁ à C₃₀ ou aralkylène en C₁ à C₃₀, les radicaux étant substitués par au moins deux atomes de fluor et en outre éventuellement substitués par OH-, un groupe alcoxy en C₁ à C₄, phényle, naphtyle, halogéno, CN-, SR₇-, NR₈R₉- et/ou -O(CO)R₁₀- ; et le radical A₁ est ininterrompu ou interrompu par un ou plusieurs -O-, -S- ou -NR₁₂-.

2. Composition photopolymérisable selon la revendication 1, dans laquelle
R représente un radical de formule II dans laquelle R₁, R₂, R₃, R₄ et R₅ représentent, indépendamment les uns des autres, un atome d'hydrogène ou un groupe alkyle en C₁ à C₄ ou alcoxy en C₁ à C₄ ;
Y représente une liaison simple,
A si s vaut 1, représente un radical A₀, A₀ représentant un groupe alkyle en C₈ à C₃₀ substitué par sept atomes de fluor ou plus et éventuellement en outre substitué par OH.
A si s est supérieur à 1, représente un radical A₁ ; A₁ représentant un groupe alkylène en C₈ à C₃₀ substitué par sept atomes de fluor ou plus et éventuellement en outre substitué par OH.

3. Composition photopolymérisable selon la revendication 1, dans laquelle
R représente un groupe phényle,
Y représente une liaison simple ;
A si s vaut 1, représente un radical A₀, A₀ représentant -(CH₂)₂-(CF₂)₅-CF₃, -(CH₂)₂-(CF₂)₆-CF(CF₃)₂, -CH₂-(CF₂)₆-CF₃, -CH₂-(CF₂)₇-CF₃, -(CH₂)-(CF₂)₁₂-CF₃, -CH₂-(CF₂)₅-CHF₂, -CH₂-(CF₂)₇-CH₂-OH ;
A si s est supérieur à 1, représente un radical A₁ ; A₁ étant : -CH₂-(CF₂)₇-CH₂-, -CH₂-(CF₂)₈-CH₂-, -CH₂-CH(CH₂-C₆F₁₃)-.

4. Composition selon l'une quelconque des revendications 1 à 3, comprenant en plus des composants (A) et (B) au moins un composé thermiquement réticulable (C).

5. Composition selon la revendication 4, comprenant en plus des composants (A) et (B), ou (A), (B) et (C), d'autres additifs (D) et/ou des photoamorceurs supplémentaires (E).

6. Procédé pour produire des revêtements ayant des surfaces stables résistant à la rayure, dans lesquels :
(1) une composition photodurcissable selon l'une quelconque des revendications 1 à 3 est préparée ;
(2) cette composition est appliquée sur un substrat ; et
(3) la composition est durcie soit seulement par exposition à un rayonnement électromagnétique avec une gamme de longueurs d'onde à partir de 200 nm dans la zone IR, soit par exposition à un rayonnement électromagnétique dans une gamme de longueurs d'onde à partir de 200 nm dans la zone IR et une exposition préalable, simultanée et/ou ultérieure à la chaleur.

7. Utilisation de compositions selon l'une quelconque des revendications 1 à 3 pour des mises en oeuvre comme le verre, telles que les fenêtres, les écrans et les fibres de verre ; pour des polymères tels que le polyester, le poly(chlorure de vinyle), le polyéthylène et le polypropylène ; le polycarbonate ; les tissus tissés et non tissés ; le papier, le verre et les métaux, tels que l'acier et l'aluminium ; les revêtements pour les lampes frontales et les revêtements en fibre de verre.
